# EUROPEAN PATENT APPLICATION

(11) **EP 2 399 929 A2**
(43) Date of publication of application: **28.12.2011**
(21) Application number: 10743985.3
(22) Date of filing: 23.02.2010
(51) Int. Cl.: C07K 14/415, C12N 15/29, A01H 1/00, C12N 15/63

(54) **POLYPEPTIDE HAVING FUNCTION FOR DELAYING ANTHESIS OR SUPPRESSING GROWTH, POLYNUCLEOTIDE ENCODING THE SAME, AND USE THEREOF**

(30) Priority: 23.02.2009 KR 20090014883
(71) Applicant: Postech Academy-industry Foundation, Gyeongsangbuk-do 790-784 (KR)
(72) Inventor: NAM, Hong Gil, Pohang-si Gyeongbuk 790-751 (KR); PARK, Kyung Mok, Pohang-si Gyeongbuk 790-752 (KR); LEE, Dong Hee, Busan 614-790 (KR); KIM, Jeong Sik, Phohang-si Gyeongbuk 790-784 (KR); LIM, Pyung Ok, Jeju-si Jeju-do 690-029 (KR)
(74) Representative: Betten & Resch
(86) International application number: PCT/KR2010/001112
(87) International publication number: WO 2010/095910

(57) **Abstract**

The present invention discloses a polypeptide having a function for delaying anthesis or suppressing growth, a polynucleotide encoding the same, and a use thereof. Specifically, the preset invention discloses a polypeptide having a function of delaying anthesis or suppressing growth, a polynucleotide encoding the same, a method for manufacturing plants with a phenotype of anthesis delay, a method for manufacturing plants with a phenotype of growth suppression, a selecting transgenic plants, and a method for screening materials which delay anthesis or suppress growth of plants.

## Description

### [Technical Field]

The present invention relates to a polypeptide that regulates a gibberellin signaling pathway, functioning to delay flowering time and to suppress plant growth, a polynucleotide encoding the same, and uses thereof.

### [Background Art]

Gibberellin, one of various hormones present in plants, was first isolated from the fungal strain *Gibberella fujikuroi,* a pathogen causing a backanae disease in rice.

In plants, gibberellin (GA) interacts with auxin to promote the elongation of young stem tissues, activated α-amylase in seeds to facilitate germination and is involved in breaking dormancy. Also, GA may be used to control flowering time. For example, plants requiring low temperatures or photoperiodism are induced to form floral buds when treated with GA. Specifically, GA is known to promote fruit setting and fruit growth even though the plants do not undergo pollination. Therefore, GA is applied to a wide range of plants in agriculture and floriculture, showing various effects including, for example, flowering in substitution for low temperature in autumn barley, peach, pear, apple and grapes, the formation of floral buds in composite plants, flowering in summer chrysanthemum, cyclamen and primrose, germination in spinach and tobacco, the breaking of dormancy in eggplant, burdock, radish and rape, and the biosynthesis of amylase, useful four the production of beer and malt. In addition, when treated with ancyumidol (A-Rest) or paclobutrazol (Bonzi), which is inhibitory of the biosynthesis of GA, plants become small and solid so that they are unlikely to be blown over by winds or a storm.

Recently, efforts have been made to study the physiological phenomena of GA on molecular levels. In the early stage of such research, variants of the dwarfism (reduced height (rth), dwarf-1 (dl), anther earl (An1)-corn; semi-dwarf(sd-1), Dwarf1, dl-qu; ls, le**,** na-bean), which led to the green evolution, or overgrowing variants (la cry³ -bean; procera(pro)-tomato; slender(sln)-barley; SLN-bean) were analyzed to deepen the study on the response of plants to GA. After 2000, as these genes were cloned (ruth (Peng et al., 1999, Nature 400:256-261), dl (Spray et al., 1996, PNAS 93:10515-10518), anl (Bensen et al., 4995, Plant Cell 7:75-84), sd-1 (Sasaki et al., 2002, Nature 416:701-702), dwarf (Ashikari et al., 1999, PNAS 67:11638-11643), el (Lester ea al., 1997, Plant Cell 9:1435-1443), na (Davidson et al., 2003, Plant Physiol. 131:335-344)), genes implicated in the synthesis, recognition and reaction of GA started to be explored. These studies have been further systemically developed using molecular and genetic methodology on Arabidopsis thaliana as a model.

Particularly, mass screening for the response of mutants to GA (dwarf mutants with low GA response, mutants that return to a wild-type upon treatment with GA, sender mutants with constitutive GA response) led to the finding of genes involved in the synthesis of GA (GA1, GA4, GA5 etc.), in the signaling pathway of GA (heterotrimeric G ptotein, GAI, RGA, SPY, SLY, etc.), and the response of GA (GA-MYB, α-amylase). In addition, the GA hormone receptor, recently found in rice (GID1:Uefuchi-Tanaka et al., 2005, Nature 437:693-698), has greatly contributed to the understanding of the molecular mechanism of GA. Currently, efforts have been continued to reveal the interrelationship between these genes, so that the GA singling pathway can be comprehensively and systemically understood on a molecular level (Sohwechheimer et al., 2008 Curr. Opin. Plant Biol., 10:461-465)

Also, GA is known to play an important role in anthesis control. The flowering time of Arabidopsis thaliana is regulated by a correlation between external signals such as light, temperature, photoperiod, etc. and internal signals such as nutrition, hormones, etc. In detail, there are four pathways known to regulate anthesis: the photoperiodic pathway, autonomous pathway, vernalization, and gibberellin (GA) pathway (Mouradov et al., 2002, Plant Cell, 14: S111-130) Particularly, the GA-deficient mutant gal does not bloom under a single condition (Wilson et al., 1992, Plant Physiol. 100:403-408), and thus GA is thought to play an important role in controlling flowering time under a single condition. The molecular mechanism underlying GA regulation of flowering time is the activation of the target genes flowering promoting factorl (FPF1) (Kania et al., 1997, Plant Cell 9:1327-1337), GA-MYB (Gocal, et al., 2001, Plant Physiol., 127:1682-1693) and SOC1 (Moon et al., 2003, Plant J., 35:613-623) to increase the transcriptional activity of LFY (Blazquez, et al., 1998, Plant Cell 10:791-800).

It is very important in crop breeding to regulate adult size and flowering time. Growth regulation gave rise to such a great increase in crop harvest as to bring about the green evolution in Asia in the 1960s. Also, growth regulators are used in Europe to prevent plants from being damaged from wind and low temperature. Hence, growth regulation is closely related with the crops of farm products. In addition, the regulation of flowering time makes it possible to determine harvest time irrespective of the time of the year and to develop species that have a harvest time that is independent of environmental factors. For this reason, those in the bioengineering field have put a great effort into regulating flowering time by genetic manipulation.

Given this background, the present invention was made.

### [Disclosure]

### [Technical Problem]

It is therefore an object of the present invention to provide a polypeptide functioning to delay flowering and/or to suppress plant growth.

It is another object of the present invention to provide a polynucleotide encoding the polypeptide.

It is a further object of the present invention to provide a method for producing a plant having a delayed flowering phenotype.

It is still a further object of the resent invention to provide a method for producing a plant having a suppressed growth phenotype.

It is still another object of the present invention to provide a method for selecting a transgenic plant using the polynucleotide.

It is yet a further object of the present invention to provide a method for screening for a delay in flowering or for a growth suppressing inducer.

### [Technical Solution]

In accordance with an aspect thereof, the present invention pertains to a polypeptide having the function of delaying flowering and/or suppressing growth (dwarfism) in plants.

As will be elucidated in the following Examples, an Arabidopsis thaliana transformant having a delayed flowering phenotype and/or a suppressed growth phenotype was produced and selected using an activation tagging method (Weigel et al., 2000, Plant Physiology, 122:1003-1013; the content of which is herein incorporated by reference in its entirety) . A gene responsible for the flowering delay and/or growth suppression in the Arabidopsis thaliana transformant was cloned using TAIL-PCR (Thermal Asymmetric Interlaced Polymerase Chain Reaction; Liu et al., 1995, Plant J. 8: 457-463; the content of which is herein incorporated by reference in its entirety) and was identified as having the nucleotide sequence of SEQ ID NO: 2 encoding the amino acid sequence of SEQ ID NO: 2. RNA gel blot was also used to determine whether the delayed flowering and/or suppressed growth phenotype results from the overexpression of the gene. Finally, when a recombinant expression vector carrying the gene was introduced thereinto, Arabidopsis thaliana was found to show the same delayed flowering and/or suppressed growth phenotype as that of the transgenic plant produced by the activation tagging method.

In detail, the polypeptide having the function of delaying flowering and/or suppressing growth in plants is one of the following polypeptides:
(a) a polypeptide having the entire amino acid sequence of SEQ. ID. NO. 2;
(b) a polypeptide containing a substantial part of the amino acid sequence of SEQ. ID. NO. 2; and
(c) a polypeptide substantially similar to that of (a) or (b).

As used in the foregoing and the following descriptions including the claims, the phrase "function for inducing flowering delay and/or suppressing growth" is intended to mean a function responsible for the expression of a delayed flowering and/or suppressed growth phenotype when the gene of the present invention (e.g., the gene having the nucleotide sequence of SEQ ID NO: 1) is overexpressed.

As used in the foregoing and the following descriptions, including the claims, the phrase or term "a polypeptide containing a substantial part of the amino acid sequence of SEQ. ID. NO. 2" is defined as a polypeptide containing part of the amino acid sequence of SEQ. ID. NO. 2, which is long enough to still have the same function, essential for flowering delay and/or growth suppression, as the polypeptide consisting of the amino acid sequence of SEQ. ID. NO. 2. Any polypeptide, as long as it retains the essential function of flowering delay and/or growth suppression, satisfies the requirement of the present invention, and thus its length or degree of activity (e.g., flowering delay and/or growth suppression) is not important. That is, even if it is lower in activity than the intact polypeptide of SEQ. ID. NO. 2, any polypeptide that has the essential function of delaying flowering and/or suppressing growth may be included within the range of "the polypeptide that contains a substantial part of the amino acid sequence of SEQ. ID. NO, 2," irrespective of the sequence length thereof. Those who are skilled in the art, that is, those who understand the prior art related to the present invention, expect that a deletion or an addition mutant of a polypeptide containing the amino acid sequence of SEQ. ID. NO. 2 will still retain the flowering delay and/or growth suppression function. As such, a polypeptide that contains the amino acid sequence of SEQ. ID. NO. 2, but from which an N- or C-terminal region has been deleted, is still functional. It is generally accepted in the are that even if its N-terminal region or C-terminal region is deleted, a mutant polypeptide can still retain the function of the intact polypeptide. As a matter of course, if the deleted N- or C-terminal region corresponds to a motif essential for the function of the peptide, the deleted polypeptide loses the function of the intact polypeptide. Nonetheless, the discrimination of such inactive polypeptides from active polypeptides is well known to those skilled in the art. Further, a mutant polypeptide which lacks a portion other than an N- or C-terminal region ran still retain the function of the intact polypeptide. Also, those skilled in the art can readily examine whether or not such a deletion mutant still retains the function of the intact polypeptide. Particularly, in light of the fact that the present invention discloses the nucleotide sequence of SEQ. ID. NO. 1 and the amino acid sequence of SEQ ID NO: 2 and further provides examples in which whether when the gene consisting of the nucleotide sequence of SEQ ID NO: 1 was overexpressed, the transgenic Arabidopsis thaliana showed a delay in flowering and/or suppressed growth phenotype was clearly examined, it will be clearly apparent that those who are skilled in the can examine whether a deletion mutant of the polypeptide comprising the amino acid sequence of SEQ. ID. NO. 2 still functions like the intact polypeptide. Accordingly, it must be understood in the present invention that "a polypeptide containing a substantial part of the amino acid sequence of SEQ. ID. NO. 2" means any deletion mutant that can be prepared on the basis of the disclosure of the invention by those skilled in the art and that retains the flowering delay or growth suppression function.

As used in the foregoing and the following descriptions, including the claims, the phase "a polypeptide substantially similar to that of (a) or (b)", means a mutant that has at least one substituted amino acid residue but still retains the function of the amino acid sequence of SEQ. ID. NO. 2, that is, the delay in lowering and/or growth suppression function. Likewise, if a mutant with at least one amino acid residue substitution still shows the flowering delay and/or growth suppression function, its activity or substitution percentage is not important. In other words, no matter how much lower a mutant polypeptide is in activity than a polypeptide containing the intact amino acid sequence of SEQ, ID. NO. 2, or no matter how much a mutant, polypeptide has been substituted with amino acid residues compared to a polypeptide containing the intact amino acid sequence of SEQ, ID. NO. 2, the mutant polypeptide is included within the scope of the present invention as long as it shows the flowering delay and/or growth suppression function. Even if it has at least one amino acid residue substituted for a corresponding residue of the intact polypeptide, the mutant polypeptide still retains the function of she intact polypeptide if the substituted amino acid residue is chemically equivalent to the corresponding one. For instance, when alanine, a hydrophobic amino acid, is substituted with a similarly hydrophobic amino acid, e.g., glycine, or with a more hydrophobic amino acid, e.g., valine, leucine or isoleucine, the polypeptide(s) containing such substituted amino acid residue(s) sill retain(s) the function of the intact polypeptide, even if it(they) has(have) lower activity. Likewise, polypeptide(s) containing substituted amino acid residue(s), resulting from substitution between negatively charged amino acids, e.g., glutamate and aspartate, still retains the function of the intact polypeptide, even if lower in activity. Also, this is true of a mutant polypeptide in which substitution occurs between positively charged amino acids. For example, a substitution mutant polypeptide, containing lysine instead of arginine, still shows the function of the intact polypeptide even if its activity is lower. In addition, polypeptides which contain substituted amino acid(s) in their N- or C-terminal regions still retain the function of the intact polypeptide. It is plainly obvious to those skilled in the art that current technology makes it possible to prepare a mutant polypeptide that retains the flowering delay and/or growth suppression function of the polypeptide containing the amino acid sequence of SEQ. ID. NO. 2, with at least one amino acid residue substituted therein. Also, those skilled in the art can examine wether a substitution mutant polypeptide still retains the function of the intact polypeptide. Further, because the present invention discloses the nucleotide sequence of SEQ ID NO: 1 and the amino acid sequence of SEQ ID NO: 2 and provides examples in which whether when the gene consisting of the nucleotide sequence of SEQ ID NO: 1 is overexpressed, the transgenic Arabidopsis thaliana shows a flowering delay and/or growth suppression phenotype was clearly examined, it will be very apparent that "the polypeptide substantially similar to that of (a) or (b)" can be readily prepared by those who are skilled in the art. Accordingly, "the polypeptide substantially similar to that of (a) or (b)" is understood to include all polypeptides that have the flowering delay or growth suppression function, in spite of the presence of at least one substituted amino acid therein.

Although "a polypeptide substantially similar to that of (a) or (b)" means any mutant that has at least one substituted amino acid residue but still retains the flowering delay or growth suppression function, a polypeptide which shares higher homology with the amino acid sequence of SEQ. ID. NO. 2 is more preferable from the point of view of activity. Useful is a polypeptide that shows 60% or higher homology with the wild-type polypeptide, with the best preference for 100% homology.

In more detail, more preferable are sequence homologies of 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% and 99.9%, in ascending order of preference. The sequence homology of 99.9% accounts for only one amino acid substitution in the polypeptide having the amino acid sequence of SEQ ID NO: 2.

Because "the polypeptide substantially similar to that of (a) or (b) includes polypeptides substantially similar to "the polypeptide containing a substantial part of the amino acid sequence of SEQ. TD. NO.2" as well as polypeptides substantially similar to "the polypeptide having an amino acid sequence entirely coincident with SEQ. ID. NO. 2", the above description is true both for polypeptides substantially similar to "the polypeptide having the entire amino acid sequence of SEQ. ID. NO. 2" and for polypeptides substantially similar to "the polypeptide containing a substantial part of the amino acid sequence of SEQ. TD. NO. 2".

In accordance with anther aspect thereof, the present invention pertains to an isolated polynucleotide encoding the above-mentioned polypeptide. Herein, the term "the above-mentioned polyeptide" is intended to include not only the polypeptide having the amino acid sequence of SEQ. ID. NO. 2, polypeptides containing a substantial part of the amino acid sequence of SEQ. ID. NO. 2, and polypeptides substantially similar to these peptides, but also all polypeptides that retain the flowering delay and/or growth suppression function in the preferred embodiments. Therefore, the polynucleotide of the present invention includes an isolated polynucleotide encoding a polypeptide that has the flowering delay and/or growth suppression function and contains the entire amino acid sequence of SEQ. ID. NO. 2 or a substantial part of the amino acid sequence thereof, and an isolated polynucleotide encoding a polypeptide substantially similar to such polypeptides. Furthermore, the polynucleotide of the present invention includes all isolated polynucleotides encoding polypeptides that share horology with the amino acid sequence of SEQ. ID. NO. 2. If an amino acid sequence is revealed, a polynucleotide encoding the amino acid sequence can be readily prepared on the basis of the amino acid sequence by those skilled in the art.

Preferable from the point of view of activity is the polynucleotide of the present invention that contains a part of the nucleotide sequence of SEQ ID NO. 1. More preferable is the entire nucleotide sequence of SEQ ID NO: 1. Herein, the phrase "a polynucleotide that contains part of the nucleotide sequence of SEQ. ID. NO. 1" means a polynucleotide teat has a sequence long enough to guarantee the flowering delay and/or growth suppression function in plants. Any polypeptide, as long as it retains the essential function of the polypeptide having the amino acid sequence of SEQ ID NO; 2, may be employed even if its activity is lower than that of the intact polypeptide, that is, the polypeptide having the amino acid sequence of SEQ ID NO: 2,

In the present invention, the phrase "the isolate polynucleotide," as used herein, is intended to include all chemically synthetic polynucleotides, polynucleotides isolated from living bodies, especially Arabidopsis thaliana, and polynucleotides containing modified nucleotides, whether single- or double-stranded RNA or DNA. Accordingly, cDNAs, chemically synthetic polynucleotides, and genomic DNAs isolated from living bodies, especially Arabidopsís thaliana, fall into the range of "the isolated polynucleotide". On the basis of the amino acid sequence of SEQ. ID. NO. 2, and the nucleotide sequence of SEQ. ID. NO. 1, encoding the amino acid sequence therefor, and technology known in the art, the preparation of corresponding cDNAs and chemically synthetic polynucleotides and the isolation of gDNA can be readily achieved by those who are skilled in the art.

In accordance with a further aspect thereof, the present invention pertains to a polynucleotide that contains or is substantially similar to part of the nucleotide sequence of SEQ. ID. NO. 1. Herein, the phrase "a polynucleotide that contains part of the nucleotide sequence of SEQ. ID. NO. 1" means a polynucleotide that has a sequence long enough to identify and/or isolate a gene having the flowering delay and/or growth suppression function in plants, especially Arabidopsis thaliana. The phrase "a polynucleotide that is substantially similar to part of the nucleotide sequence of SEQ. ID. NO. 1" means a polynucleotide that contains at least one substituted nucleotide residue, compared to the nucleotide sequence of SEQ. ID. NO. 1, and has sequence-dependent binding ability sufficient to identify and/or isolate a gene having a flowering delay and/or growth suppression function in planes including Arahidqps-is thaliana*.* As long as the nucleotide sequence of SEQ. ID. NO. 1 is disclosed, the identification and/or isolation of a gene having the flowering delay and/or growth suppression function in *Arabídopsis thaliana* or other organism can be readily carried out on the basis thereof by those skilled in the art. Accordingly, the polynucleotide of the present invention is intended to include all polynucleotides which have a sequence length or sequence-dependent binding power sufficient to identify and/or isolate a gene having the flowering delay and/or growth suppression function in plants including Arabidopsis thaliana irrespective of the length and sequence homology to the nucleotide sequence of SEQ. ID. NO. 1.

In order to be used as a probe for examining whether or not an unknown gene has the same nucleotide sequence as that of a known gene or for isolating an unknown gene, a polynucleotide is generally known to have to contain 30 or more consequent nucleotide residue. Thus, the polynucleotide of the present invention preferably includes 30 or more consecutive nucleotide residues out of the nucleotide sequence of SEQ. ID. NO. 1. Nevertheless, a poly (or oligo) peptide consisting of 30 or fewer consequent nucleotide residue out of the nucleotide sequence of SEQ. ID. NO. 1 is still included within the scope of the present invention. The reason is that the poly (or oligo) nucleotide, although short, is sufficient to identify and/or isolate a gene having the flowering delay or growth suppression function from Arabidopsis thaliana or other organisms if it shares 100% homology with part of the nucleotide sequence of SEQ. ID. NO. 1 and the identification and/or isolation conditions (buffer pH, concentration, etc.) are stringent. Based on the disclosure of the present invention herein, those skilled in the art can readily construct and detect a polynucleotide which is 30 or fewer bases long in order to identify and/or isolate a gene having the flowering delay or growth suppression function from *Arabidopsis thaliana* or other organisms, and can readily identify and/or isolate a gene having the flowering delay and/or growth suppression function from *Arabidopsis thaliana* or other organisms using the constructed polynucleotide.

As used in the foregoing and the following descriptions, including the claims, the term "plant" is intended to include all plants which produce results beneficial to humans when their biomass is decreased. The most direct examples of such plants include various weeds inhibitory of the growth of crops, potted plants, flowering plants, etc. In addition, edible plants may fall into the range of being considered plants on the grounds of the simplicity of eating them, convenience of their transportation, etc. In greater detail, the examples of the plant include weeds growing on arable lands, potted plants such as roses, pine trees, nut pines, bamboos, etc., flowering plants such as gladiola, gerberas, carnation, chrysanthemums, lilies, tulips, etc., cereal plants such as rice, wheat, barley, corn, bean, potato, adzuki bean, oats, millet, etc., vegetable plants such as Arabidopsis thaliana, Chinese cabbage, radish, pepper, strawberry, tomato, water melon, cucumber, cabbage, oriental melon, pumpkin, Welsh onion, onion, carrot, etc., industrial crops such as ginseng, tobacco, cotton, sesame, sugarcane, sugar beet, perilla, peanut, canola, fruits such as apple, pear, jujube, peach, kiwi, grape, tangerine, persimmon, plum, apricot, banana, etc., and fodder plants such as rye grass, red clover, orchard grass, alphalpha, tall fescue, perennial rye grass, etc., but are not limited thereto.

Also, the term "plant", as used herein, must be understood to include not only adult plants, but also plant cells, tissues, and seeds which can develop into adult plants.

In accordance with still a further aspect thereof, the present invention pertains to a method for producing a plant having delayed flowering and/or a suppressed growth phenotype.

The term "flowering delay" in all its grammatical forms and spelling variations means a flowering time later than that in a wild-type plant when the same culture conditions such as temperature, the period of time of light and dark, etc. are provided.

The term "growth suppression" in all its grammatical forms and spelling variations is used to mean that the biomass of a plant is less than that of the wild-type, preferably in the stems and/or leaves. Herein, biomass may be understood to indicate weight, length and/or size of plant organs, such as leaves, stems, etc.

The method for producing a plant having a delayed flowering and/or suppressed growth phenotype in accordance with the present invention comprises (I) overexpressing a gene having the nucleotide sequence of SEQ ID NO: 1 or a nucleotide sequence similar to that of SEQ ID NO: 1, and (II) selecting the plans in which delayed flowering and/or suppressed growth phenotype is induced.

As used herein, the term "overexpression" in all its grammatical forms and spelling variations means a gene expression at a level higher than that in a wild-type plant under the same culture conditions such as temperature, the period of time of light and dark, etc.

As used herein, the phrase "gene having a nucleotide sequence similar to that of SEQ ID NO: 1" is intended to refer to a homologue of the gene of the nucleotide sequence of SEQ ID NO: 1 within the scope of which all genes that have different nucleotide sequences according to plant species as a result of different evolution paths, but retain the function for flowering delay and/or growth suppression are encompassed. A gene which shares higher horology with the nucleotide sequence of SEQ ID NO; 1 is more preferable. Of course, a sequence homology of 100% is the most preferable. As for she lower limit of the horology with the nucleotide sequence of SEQ ID NO; 1, it is preferable 60%. In more detail, more preferable are sequence homologies of 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99%, in ascending order of preference.

Another preferred example of the gene having a nucleotide sequence similar to that of SEQ ID NO: 1 is a functional equivalent to the gene which arises as a consequence of codon degeneracy. The functional equivalent of the gene encompasses polynucleotides coding for a polypeptide having the amino acid sequence of SEQ ID NO: 2.

In the method of the present invention, the overexpression step (I) preferably comprises transforming a polynucleotide coding for the polypeptide of the present invention into a plant.

The term "transformation"' in all its grammatical forms and spelling variations, as used herein, means the introduction of a foreign polynucleotide (encoding a polypeptide having the function for inducing lowering delay and/or growth suppression) into a host plant and more accurately into a plant cell irrespective of the method, resulting in a genetic alteration in the host plant, Once introduced into a host plant, the foreign polynucleotide may stay integrated into the genome of the host plant or be separated, both being encompassed by the present invention.

The transformation of a plant with a foreign polynucleotide may be achieved using typical methods known in the art (Methods of Enzymology, Vol. 153, 1987, Wu and Grossman ed., Academic Press).

For the transformation, a foreign polynucleotide may be inserted into a vector, such as a plasmid or a virus, which is used as a vehicle carrying the foreign polynucleotide into plants. Further, the recombinant vector may be transformed into Agrobacterium bacteria which is used as a mediator for DNA transmission to plants (Chilton et al., 1977, Cell 11:263-271). Alternatively, a foreign polynucleotide may be introduced directly into a plant (Lorz et al., 1985, Mol. Genet. 199:178-182).

Widely used is a plant transformation method in which *Agrobacterium tumefaciens* harboring an erogenous polynucleotide is transfected into young plants, plant cells or seeds. Those skilled in the art can culture and grow the transected plant cells or seeds into mature organisms.

The transformation step is preferably carried out by (a) inserting a polynucleotide encoding the polypeptide of the present invention in an operably linking manner into an expression vector containing a regulatory nucleotide sequence to construct a recombinant expression vector and (b) introducing the recombinant vector into a host plant to afford a transgenic plant.

Preferably, the transformation step comprises inserting a polypeptide encoding the polypeptide of the present invention in an operably linking manner into an expression vector containing a regulatory nucleotide sequence to constrict a recombinant expression vector, transforming an Agrobacterium sp. with the recombinant expression vector, and transfecting the transformed Agrobacterium sp. into a plant. More preferably, the tranformed Agrobacterium sp. is transformed *Agrobacterium tumefaciens.*

The term "regulatory nucleotide sequence" must be understood to include all sequences that have an influence on the expression of the gene of interest. Examples of the regulators nucleotide sequence include leader sequences, enhancers, promoters, initiation codon, termination codon, replication origin, ribosome-binding site, etc.

The term "operably linking" or "operably linked", as used herein, is used to mean that a regulatory sequence is functionally linked to another nucleotide sequence, thereby regulating she transcription and/or translation of this nucleotide sequence. For example, if a promoter affects the transcription of a gene of interest located in the same vector, the gene is operably linked.

As for promoter sequences useful in the present invention, they may be inducible or constitutive. Representative of constitutive promoters are CaMv promoters, CsVMV promoters, and Nos promoters. Example of inducible promoters (the activity of the promoter is induced by an induce to express an operable linked gene) include a yeast-copper metallothionein promoter (Mett et al., Proc. Natl. Acad. Sci., U.S.A., 90:4567, 1993), substituted benzenesulfonamide-inducible In2-1 and In2-2 promoters (Hershey et al., Plant Mol. Biol., 17:679, 1991), a glucocorticoid response element (GRE) (Schena et al., Proc. Natl. Acad. Sci., U.S.A., 88:10421, 1991), an ethanol-inducible promoter (Caddick et al., Nature Biotech., 16:177, 1998), a light-inducible promoter from the small subunit of ribulose-1,5-bisphosphate carboxylase (ssRUBISCO) (Coruzsi et al., EMBO J., 3:1671, 1984; Broglie et al., Science, 224:838, 1984), a manopine synthase promoter (Velten et al., EMBO J., 3:2723, 1984), opaline synthase (NOS) and octopine synthase (OCS) promoters, and a heat-shook promote (Gurley et al., Mol. Cell. Biol., 6:559, 1986; Severin et al., Plant Mol. Biol., 15:827, 1990).

The recombinant vector may harbor a selectable marker gene. The term. "marker gene", as used herein, is intended to refer to a gene encoding a character which allows the selection of the plans or plant cell containing the gene. Harken genes may be resistant to antibiotic or herbicides. Examples of the selectable marker genes useful in the present invention include an adenosine deaminase gene, a dihydrofolate reductase gene, hydromycin-B-phosphotransferase gene, a thymidine kinase gene, a xanthine-guanine phosphoribosyl transferase, and a phosphinotricine acetyltransferase gene.

In an embodiment of the present invention, a gene consisting of the base sequence of SEQ ID NO. 1 is inserted into the expression vector pCambia1302 to construct a recombinant vector pCambia1302-SIL1 which is in turn transformer into *Agrobacterium tumefaciens,* followed by the transfection of the transformed *Ayrobacterium tumefaciens* into *Arabidopsis thaliana.*

When the embodiment of the present invention is taken into consideration, the transformation step preferably comprises transforming a plans with a gene consisting of the base sequence of SEQ ID NO. 1 and more preferably with a recombinant vector containing the gene, especially pCambia1302-SIL1, and most preferably transfecting Agrobacterium tumefaciens carrying pCambia1302-SIL1 into a plant.

The selection step (II) may be carried out by selecting plants with the naked eye after the growth of the transgenic or transformed plant of step (I) or by taking advantage of a selectable marker gene introduced at the same time into the plant.

In accordance with still another aspect thereof, the present invention pertains to a plant, produced by the method for producing a plant having delayed flowering and/or suppressed growth phenotype. The plans of the present invention is understood to encompass a transgenic plant in which a delayed flowering and/or suppressed growth phenotype is induced by overexpression of the gene having the nucleotide sequence of SEQ ID NO: 1 or having a nucleotide sequence similar to that of SEQ ID NO: 1.

In accordance with yet a further aspect thereof, the present invention pertains to a method for selecting a transgenic plant using the above-mentioned polynucleotide of the present invention as a marker gene.

The method for selecting a transgenic plant in accordance with the present invention comprises (I) transforming a plant with an expression vector carrying a target gene, an above-mentioned polynucleotide coding for a polypeptide able to induce flowering delay and/or growth suppression in plants, and a regulator nucleotide, and (II) discriminating a flowering delay and/or growth suppression-induced plant valiant from the non-induced once.

As used herein, the term "target gene" is defined as a polynucleotide sequence encoding a product of interest, be it natural or mutant (i.e., RNA or polypeptide). The target gene may be cDNA or gDNA in a truncated, fused or ragged form, encoding a native product or a desired mutant.

The step (I) of transforming a plans with an expression vector may be carried out by transforming the expression vector into Agrobacterium spp. and transfecting the transformed Agrobacterium sp. into the plant. The Agrobacterium sp. is preferably Agrobacterium tumefaciens. Preferably, the Agrobacterium sp. is Agrobacterium tumefaciens.

Also, a further description of the method for selecting the transgenic plants may refer to that given for the method for producing a plant having a delayed flowering phenotype.

In accordance with yet a further aspect thereof, the present invention pertains to a method for screening a planet flowering delay inducer.

In accordance with yet another aspect thereof, the present invention pertains to a method for screening a plant growth suppression inducer.

These methods comprise (I) treating a plant with a chemical or biological material, and (II) detecting the inducer which causes the expression of a gene consisting of a nucleotide sequence identical or similar to that of SEQ ID NO. 1.

The term "gene consisting of a nucleotide sequence similar to that of SEQ ID NO: 1" is as described above.

The treating step (I) may be conducted by bringing the plant into contact with a chemical or by using a bioengineering technique as described when describing the method for producing a flowering-delayed plant.

As candidates for the plant inducers, examples include the sense nucleotide sequence of SEQ ID NO. 1, a recombinant vector carrying the sense nucleotide sequence, and Agrobacterium tumefaciens transformed with the recombinant vector.

### [Advantageous Effects]

As described hitherto, a polypeptide functioning to induce flowering delay and/or growth suppression in plants, and a polynucleotide coding therefor are provided. Also provided are respective methods for producing a transgenic plant showing a delayed flowering phenotype, for producing a transgenic plant showing a suppressed growth phenotype, for electing the transgenic plants, and for screening an agent functioning to indue flowering delay or growth suppression in plants.

### [Description of Drawings]

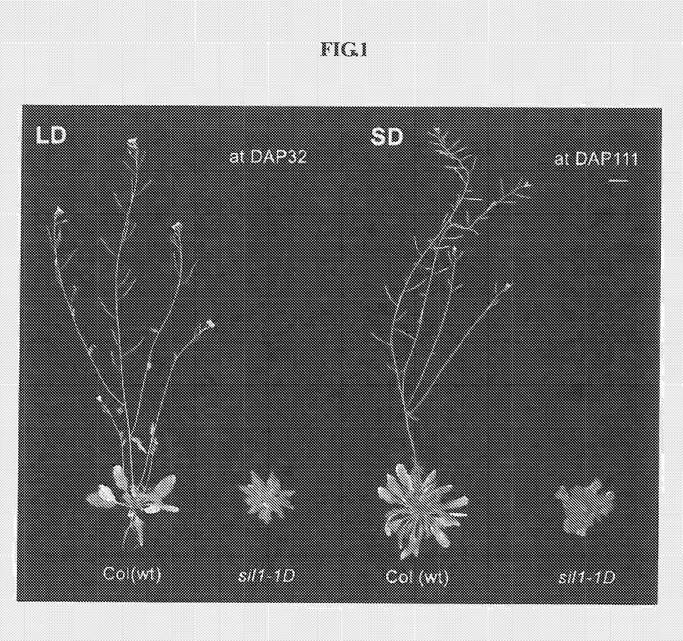

FIG. 1 is a photograph showing the time of generation of floral axis in the Arabidopsis thaliana Columbia wild-type (Co1-0(wt)) and the flowering delay-induced Arabidopsis thaliana mutant (*si11-1D*) upon cultivation under long daylight (LD)/short daylight (SD) conditions. Compared to the wild-type, as seen, the flowering of the *si11-1D* mutant was significantly delayed under LD/SD conditions. DAP (days after planting).

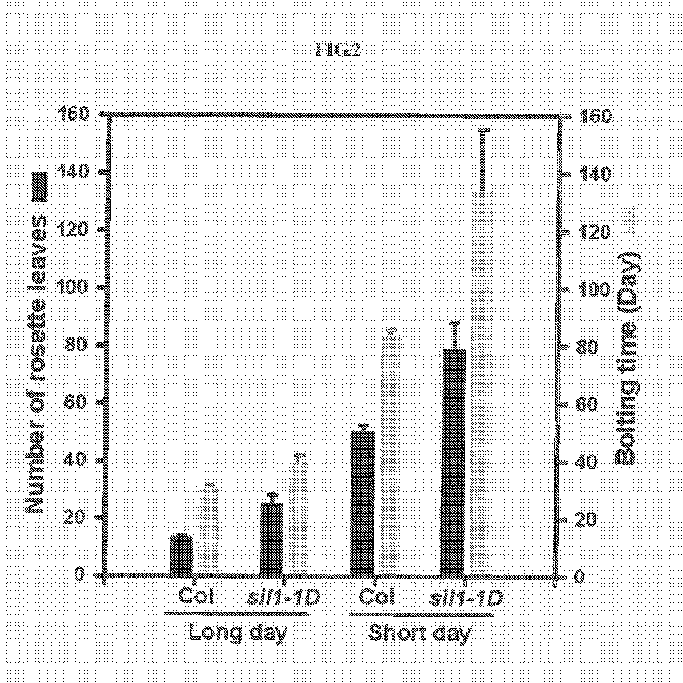

FIG. 2 is a graph showing the results of FIG. 1. In the graph, the numbers of rosette leaves upon flowering time are shown according to plants cultured under long day and short day conditions. Also, bolting time, which is the time taken to bloom after seeding, was also depicted.

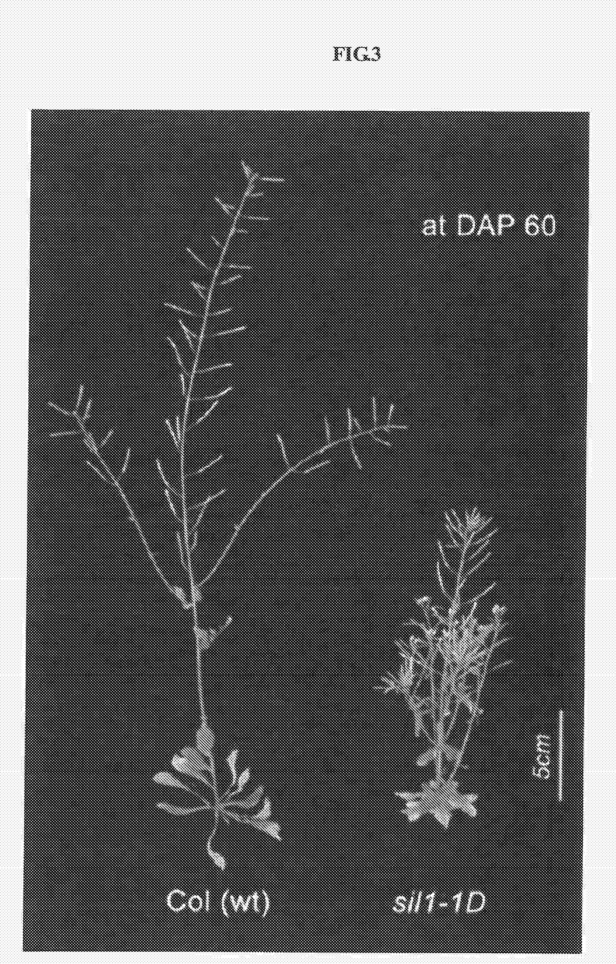

FIG. 3 is a photograph showing the adult sizes of the wild-type and the *si11-1D* mutant. The height of the *si11-1D* mutant is half that of the wild-type when they were completely grown.

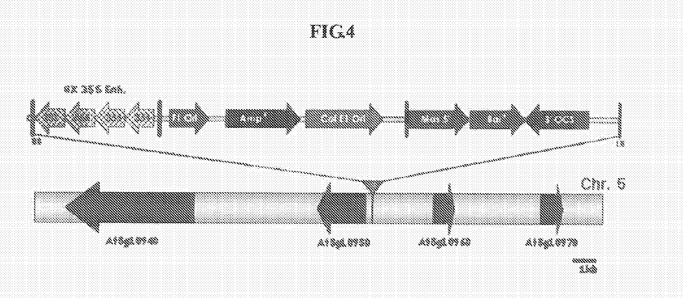

FIG. 4 is a schematic diagram of the activation tagging vector pSKI015 integrated into the genome of the transgenic Arabidopsis thaliana exhibiting a delayed flowering and/or suppressed growth phenotype. p5KI015 is inserted upstream of At5g10950 in the genome of Arabidopsis thaliana. In she schematic diagram, 4x35S Enh stands for four consecutive 35S enhancers, Bar^{R} for a gene conferring to Basta herbicide resistance, Amp^{R} for ampicillin-resistant gene, and Col E1 Ori for E. coli replication origin.

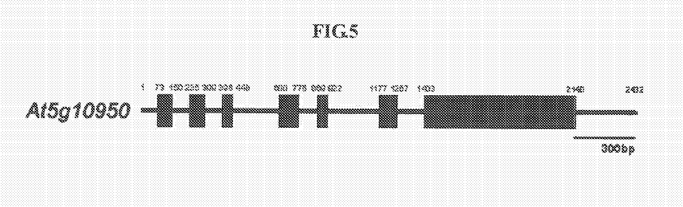

FIG. 5 shows a structure of the At5g10950 gene of Arabidopsis thaliana. The gene comprises 7 exons consisting of 1185 nucleotide residues, and 6 introns.
FIG, 6 shows the amino acid sequence of the protein encoded by At5g10950 of Arabidopsis thaliana. The protein consists of 395 amino acids, with putative nuclear localization signals boxed. They were determined by computer-aided sequence analysis.

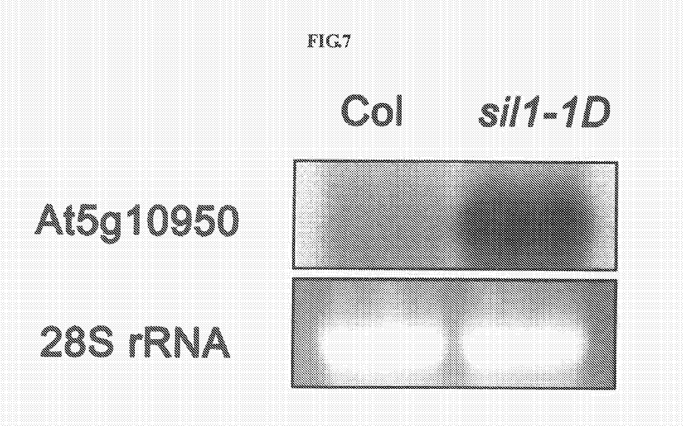

FIG. 7 shows the expression patterns of the At5g10950 gene located in the vicinity of T-DNA in the wild-type and the *si11-1D* mutant as analyzed by RNA gel blotting. 28S rRNA was used as a control. The expression level of At5g10950 was significantly increased in the *si11-1D* mutant.

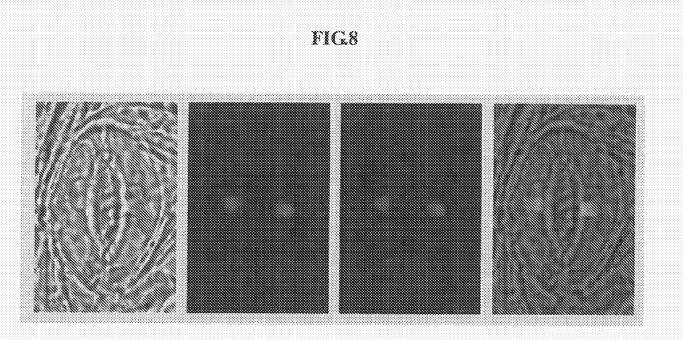

FIG. 8 shows the expression position of SIL1 in guard cells of the Arabidopsis thaliana transformed with pCambia 1302-SIL1. An optical micrograph of a guard cell is given in the first panel. Green fluorescence derived from SIL1 and GFP fusion protein is shown in the second panel. Blue fluorescence shows nuclei stained with DAPI in the third panel. These three panels are overlapped to give a complex image of green and blue fluorescence in the fourth panel.

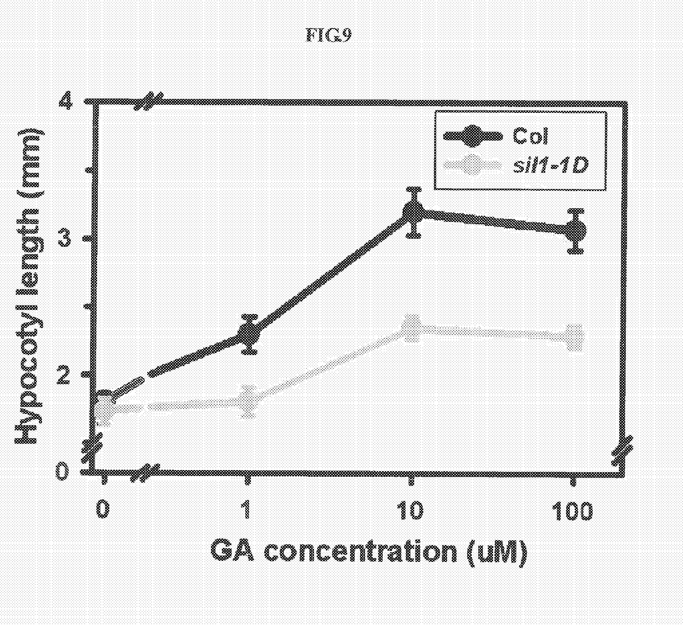

FIG. 9 is a graph showing hypocotyl growth in the wild-type and the *si11-1D* mutant in response to various concentrations of gibberellin (GA). The hypocotyl length of the wild-type increased with increasing GA concentration. The GA-induced hypocotyl growth of the *si11-1D* was significantly reduced, compared to the wild-type.

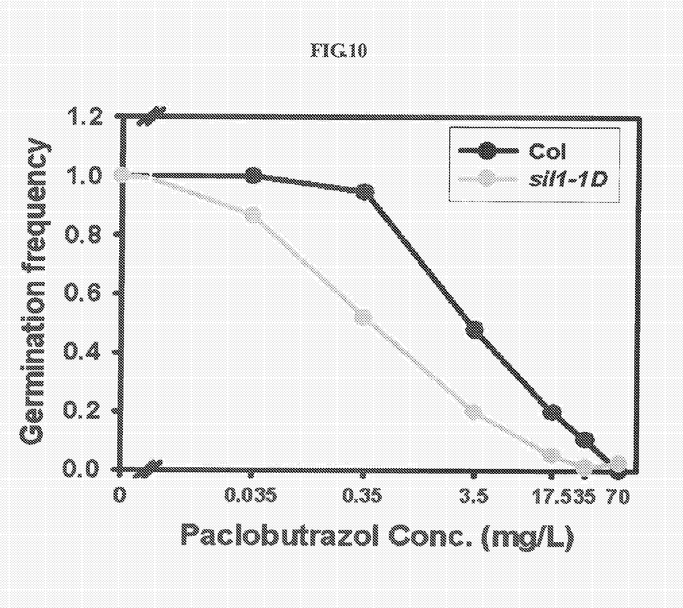

FIG. 10 is a graph showing germination efficiencies of the wild-type and the *si11-1D* mutant when they are treated with Paclobutrazol (PAC), a GA biosynthesis inhibitor. Germination efficiency of both the wild-type and the *si11-1D* mutant decreased with increasing of PAC concentration, with a higher PAC-induced germination inhibition affect given to the *si11-1D* mutant.

### [Mode for Invention]

### <EXAMPLE 1> Preparation and Selection of Arabidopsis thaliana Transformant Showing Delayed Flowering and/or Suppressed Growth Phenotype

An Arabidopsis thaliana transformant showing a delayed flowering and/or suppressed growth phenotype was prepared and selected according to the method of Weigel et al. (Heigel et al., 2000, Plant Phtsiology, 122:1003-1013).

The activation tagging vector pSK1015 (Weigel et al., 2002, Plant Physiol., 122; 1003-1013: granted from the Weigel Lab. Germany), as seen in FIG. 4, which contains four CaMV 35S enhancers located at the right border of T-DNA and a *bar* gene (phophinithricin acetylltransferase gene) conferring to Basta resistance, was introduced into *Agrobacterium tumefaciences* strain A8I (Lazo et al., 1991, Biotechnology 9:963-967; granted from Amassino Lab. U.S.A.) by electroporation, followed by selecting the transformant on a medium containing kanamycin and carbenicillin. Subsequently, the Agrobacterium strain transformed with pSK1015 was transfected into Arabidopsis thaliana Columbia wild-type using a floral dipping method (Clough et al., 1998, Plant J., 16:735-743). The transformed *Arabidopsis* thaliana strains were cultured in the presence of a Basta herbicide to obtain seeds which were then grown at 23 °C in a greenhouse to 5,000 T1 lines. Out of them, one line which was observed to have a delayed flowering and suppressed growth phenotype as measured with the naked eye was selected, and named *si11-1D* (short internode and lathe flowering1). Thereafter, the selected transgenic Arabidopsis thaliana (*si11-1D*) and the Arabidopsis thaliana Columbia wild-type (Col(wt)) were cultured at 23°C in respective growth chambers with dark/light cycles of 16/8. As can be seen in FIGS. 1 and 2, the transgenic Arabidopsis thaliana *si11-1D* exhibited a delayed flowering phenotype, characterized in that the plant flowered at a later time, with more leaves at flowering time, under both long- and short daylight, compared to the wild-type. Also, the growth of *si11-1D,* as shown in FIG. 3, was suppressed so that its height became as short, as half of that of the wild-type.

### <EXAMPLE 2> Identification of Overexpressed Gene in the Transgenic Plant

TAIL-PCR (Liu et al., 1995, Plant J. 8: 457-463) was performed to clone a gene responsible for the delayed flowering and/or suppressed growth phenotype in the selected transgenic Arabidopsis thaliana of Example 1.

For this PCR, three arbitrary primers (AD1, AD2, AD3) having respective nucleotide sequences of SEQ ID NOS: 3 to 5 and three specific primers (AT1, AT2, AT3) having respectively nucleotide sequences of SEQ ID NOS: 6 to 8 were used, PCR was carried out in the presence of Takara Extaq in 2700 thermocyclers, Applied Biosystems.

TAIL-PCR was comprised of three-step reactions, each being performed under the following conditions.

### (first step PCR)

In this PCR, the genomic DNA extracted from the transgenic Arabidopsis thaliana of Example 1 (Dellaporta et al., 1983, Plant Mol. Biol. Rep. 1:19-21) was used as a template and the total volume of the reaction was set to 25 µL. Each PCR reaction was carried out in three tubes (AD1, AD2, or AD3). In the first-step of PCR, the final concentrations of ingredients and the number and conditions of reaction cycles are as follow.

### (Final concentration)

Template DNA; 100 ng
AT1 0.2 µM
AD1, AD2, or A3 primer 2 µM
dNTP mix: 0.8 mM
Taq enzyme: 0.5 U

### (first-step PCR reaction cycle)

PCR started with one cycle of 93°C for 1 min and 95°C for 1 min and was performed with five cycles of 94°C for 30 sect 62°C for 1 min and 72°C for 2.5 min, followed by 94°C at 30 sec and 25°C for 3 min. Then, the temperature was gradually increases to 72 °C over a period of three min or longer and maintained at 72°C for 2.5 min, after which 15 cycles of 94 °C for 10 sec, 68°C for 1 main, 72°C for 2.5 min, 94°C for 10 sec, 68°C for 1 min, 72°C for 2.5 min, 94°C for 10 see, 44°C for 1 min, and 72°C for 2.5 main was repeated, followed by 72°C for 1 min.

### (Second-step PCR)

For use as a template in this second-step of PCR, one pal taken from 50 µL of the first-step PCR redaction mixture was 1/50 diluted in 49 µL of sterilizes water. The same arbitrary primers as in the first-step reaction were also employed and the total volume of the reaction was set to be 25 µL. In the second-step PCR, the final concentrations of ingredient and the number and conditions of reaction cycles are as fellows.

### (Final Concentration)

Template DNA: 1 µL
AT2 0.2 µM
AD1, AD2, or AD3 primer: 2 µM
dNTP mix: 0.8 mM
Tag enzyme: 0,5 U

### (Number and conditions of reaction cycle)

PCR was performed with 12 cycles of 94°C for 10 sec, 64°C for 1 min, 72°C for 2.5 min, 94°C for 10 sec, 64°C for 1 min, 72°C for 2,5 min, 94°C for 10 sec, 44°C for 1 main and 72°C for 2.5 main, followed by 72°C for 1 min.

### (Third-step PCR)

In this PCR, 1 µL taken from 25 µL of the second-step PCR reaction mixture was 1/10 diluted in 9 µL of sterile water and used as a template, The same arbitrary primers as in the first-step reaction were also employed and the total volume of the reaction was set to be 50 µL. In the third-step PCR, the final concentrations of ingredients and she number and conditions of reaction cycles are as follows

### (Final concentration)

Template DNA: 1 µL
AT 3 0.2 µM
AD1, AD2, or AD3 primer: 2 µM
dNTP mix: 0.8 mM
Taq enzyme: 1 U

### (Number and conditions of reaction cycles)

PCR was performed with 20 cycles of 94 °C for 15 sec, 44°C for 1 min and 72°C for 2.5 main, followed by 72°C for 1 min,

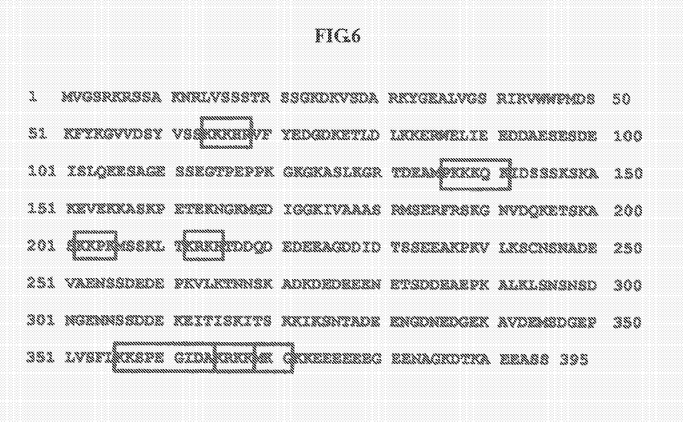

After conviction of the three-step PCR, thy PER product thus obtained was subjected to agarose gel elect-ophoresis and isolated from the gel. Its nucleotide sequence was determined using a sequencer (ABI3730; Applied Biosystem Inc.). On the basis of the determine nucleotide sequence, an open reading frame nearest to the enhances was discovered from she genome database of Arabidopsis thaliana. The gene was found to be inserted at the vicinity of the At5g10950 promoter in the Arabidopsis thaliana genome, as shown in FIG. 4. The gene comprises seven exons (FIG. 5) and consists of 1185 nucleotides, encoding 395 amino acids (FIG. 6). Herein, the gene and the protein were named *SIL1* gene and SIL1 protein, respectively.

### <EXAMPLE 3> Assay of Overexpression of SIL1 Gene by RNA Gel Blot

To examine whether the transgenic Arabidopsis thaliana (*sil1-1D*) overexpressed *SIL1* gene, RNA gel blotting was performer, with a *SIL1* cDNA fragment serving as a probe. Total RNA was isolated from the Arabidopsis thaliana Columbia wild-type (Col-0 (wt)) and the transgenic Arabidopsis thaliana *sil1-1D* prepared in Example 1 using TRIzol-Reagent (Invitrogen) according to the manufacturer's instruction. Then, 30 µg of each of the RNA extracts was separated on 1.2% formaldehyde-agarose gel and transferrer to a nylon membrane (Hybond N⁺, GE healthcare Bioscienoe) using a vacuum transport system (GE healthcare Bioscience). The nylon membrane was exposed to UV on a do-crosslinker (Stratagene) and dried at 65°C for 1 hour. The tried nylon membrane was treated with radio-labeled probes and hybridized at 65°C for 16 hours in a church and Gilbert solution (25mM sodium phosphate, 1mM EDTA, 7% SDS, 1% BSA). The probe was synthesized from Arabidopsis thaliana cDNA by PCR using a forward primer of SEQ ID NO: 9 (0.2 µM) and a reverse primer of SEQ ID NO; 10 and dNTP (each 2.5mM) in GeneAmp 2700 (Applied Biosystems). After starting at 95°C for 3 min for denaturing, PCR was performed with 35 cycles of denaturing at 94°C for 15 sec, annealing at 53°C for 30 spec and elongation at 72°C for 1.5 min, followed by elongation at 72°C for 7 min. The PCR product was electrophoresed in 1 % agarose gel for 1 hour at 100 V and the *SIL1* gene was elated from the gel using a gel elation kit (Qiagen). This DNA was labeled with 5 µL dCTP (1 mCi/µL, PerkinElmer) using a random priming kit (GE healthcare Bioscience, USA) according to the manufacturer's manual. After the hybridization, the nylon membrane was washed at 65°C for 10 min with a solution containing 2x *SSC* (sodium chloride/Sodium Citrate) and 0.2% SDS (Sodium dodecyl sulfate), for 10 min with a solution containing 1x SSC and 0.1% SDS, and finally with a solution containing 0.1x sac and 0.1% SDS. The washed nylon membrane was exposed for two days to a BAS film and analyzed using a phosphorimager (BAS2000, Fuji, Japan). The result is shown in FIG. 7.

As can be seen from FIG. 7, *SIL1* mRNA expression is extremely low in Columbia while it is expressed at a significantly high level in the transgenic Arabidopsis thaliana (*sil1-1D*).

### <EXAMPLE 4> Construction of SIL1-Carrying Expression Vector and Transformant, and Intracellular Expression

To determine whether the phenotype of the transgenic Arabidopsis thaliana produced in Example 1 resulted from the overexpression of *SIL1* gene, first, the *SIL1* gene was inserted into pCambia 1302 (Caberra, Australia; Hajdukiewicz et al, 1994, Plant Mol. Biol. 25:989-994) and expresses. In this retard, the SIL1 probe DNA amplified from Arabidopsis thaliana cDNA in Example 3 was subcloned into T vector using a GEM T easy vector kit (Promega). Then, 2 µg of she clone was digested with 2 µL (10 U/µL) *Nhe*I and *Spe*I while 2 µg of pCambia 1302 was treated with 2 µL (10 U/µL) at 37°C for 2 hours. Using a gel elution kit, a 1.19 kb DNA fragment of the *SIL1* gene and a 10.5 kb fragment of pcc-mbia 1302 were purified from the gel, and ligated together at 16°C for 6 hours in the presence of 1 µL (1 µg/µL) of T4 lipase (Roche). The recombinant vector carrying the identified *SIL1* gene (named pCambia 1302-SIL1) was introduced into *Agrobacterium tumefacience* AGL1 strain using electroporation, followed by selection in a medium containing kanamycin. The Agrobacterium strain harboring the pCambia 1302-SIL1 clone was transected into an Arabidopsis thaliana Columbia wild-type using a floral dipping method (Clough et al., Plant J., 16(6):735-743, 1998). Thereafter, seeds from the transected Arabidopsis thaliana were grown in a medium containing 15 µg/ml hygromycin to select transgenic plants. The transgenic Arabidopsis thaliana was incubated in a growth chamber and analyzed for flowering delay and growth suppression pattern The same delayed senescence phenotype as shown in the *sill-1D* mutant was reproduced, indicating that the delayed flowering and suppressed growth phenotype is attributed to the activation of the *SIL1* gene. Leaves were taken from one line of the transgenic plants, treated with 1 µg/µL DAPI (4', 6-diamidino-2-phenylindole), and observed under a fluorescence microscope (Axio Vert 200; Carl Zeiss, Gottingen). Fluorescence signals of SIL1-GFP (for green fluorescence protein) demonstrated the vocalisation of the protein within the nucleus, like DAPI, suggesting that SIL1 acts as nuclear factor.

### <EXAMPLE 5> Assay of Response of sill-1D Mutant to GA with Regard to Hypocotyl Length

Under both long and short daylight conditions, the *sil1-1D* exhibited delayed flowering and suppressed growth, with dark green leaves, as described in Example 1. In most cases, these phenomena occur when the plant becomes low in GA response or GA biosynthesis level. Thus, the response of *sil1-1D* to GA was examined. In this regard, hypocotyl lengths of 15 plantlets were measured after they were grown in 1/2 Gamborg B5 media (Duchefa) containing 1% sucrose and various concentrations of GA under white light As can be seen in FIG. 9, the hypocotyl growth of the wild-type (Col) increased with increasing of GA concentration under white light. In contrast, *sil1-1D* had short hypocotyls in response to GA, compared so the wild-type, which indicates that the response of *sil1-1D* to GA is lowered in terms of hypocotyl growth.

### <EXAMPLE 6> Assay of Response of sil1-1D Mutant to paclobutrazol with regard two germination

It is generally known that GA is involved in germination as well as hypocotyl growth as shown in Example 5. Hypocotyl growth or adult heights are associated with cell elongation. Typically, GA, auxin and brassinosteroid (BA) are involved in cell elongation while plant hormones such as GA and ABA are responsible for germination. As for she response to GA, its molecular mechanism are different for different phenomena. Thus, in order to support the fact that the delayed flowering and/or dwarfism phenotype is attributed to the deficient response of *sil1-1D* to GA, the wild-type (Col) and *sil1-1D* were treated with paclobutrazol (PAC), which inhibits the biosynthesis of GA, and their germination efficiencies were compared. When treated with 0.35 mg/L PAC, as shown in FIG. 10, the wild-type sowed almost 100% germination, but the germination of *sil1-1D* remained at about 50%. Generally, whey GA biosynthesis was inhibited by PAC, germination efficiency was glowered, with a lowered response to GA. This indicates that the response of *sil1-1D* to GA is lowered in terms of germination effect. Taken together, the data demonstrate that the overexpression of SIL1 has an influence on GA response, causing growth suppression and flowering delay.

### [Sequence List Text]

**Attached**

<110>    POSTECH ACADEMY-INDUSTRY FOUNDATION

<120>    Polypeptide Having a Function of Delaying Flowering or
         Suppressing Growth, Polynucleotide Encoding the Polypeptide and
         Uses Thereof

<160>    10

<170>    KopatentIn 1.71

<210>    1
<211>    1188
<212>    DNA
<213>    Arabidopsis thaliana

<400>    1
atggttggtt ctaggaagcg ttcatcagcc aagaacagac ttgtttcttc ttcaactcgt
60

tcttctggga aggataaggt ttctgatgct cgtaaatatg gtgaagcctt ggtgggttca
120

aggatacgag tttggtggcc gatggacagc aaattctata agggtgtggt ggattcctat
180

gtctcttcca agaagaaaca tcgggttttc tatgaagatg gtgataaaga gaccttggat
240

ctgaagaagg agagatggga gttgattgaa gaagatgatg ctgaatctga atctgatgag
300

atctccctac aggaggaatc tgctggtgag agttctgaag gcactccaga accgcctaaa
360

gggaaaggga aggcatcttt aaaaggaagg acggatgaag ctatgcccaa aaagaaacag
420

aagatagatt cttcatcaaa gagcaaggcc aaagaggtgg agaagaaggc ctcaaagccc
480

gaaactgaga aaaatggcaa aatgggtgat attggtggga agattgtagc agcggcatca
540

cgcatgagtg aaaggtttcg gagcaagggg aatgtggatc aaaaagagac atcaaaggcc
600

tcgaaaaaac caaagatgtc ttcaaagttg accaagagga aacacactga tgaccaagat
660

gaagatgaag aagctggtga tgatattgat acttctagcg aagaggcgaa gcctaaggtg
720

ttaaaatcct gcaactccaa tgctgatgaa gttgctgaaa attcttctga tgaagatgag
780

cccaaggttc taaaaaccaa caactctaag gctgataaag atgaagatga agaggagaat
840

gaaacatctg atgatgaggc tgagcccaag gccctgaaat tgagcaactc caactctcat
900

aatggtgaaa acaattcatc tgatgatgag aaagagataa caatatcgaa gatcacctca
960

aaaaagatta agagtaatac cgctgatgaa gaaaatggtg acaatgaaga tggggaaaaa
1020

gctgttgatg aaatgtctga tggagagccg ttggtgagct tcttgaagaa gtcaggagag
1080

ggaattgatg cgaagaggaa aaaaatgaag gggaagaagg aggaagagga agaggaagga
1140

gaagaaaatg ctggaaaaga tacgaaagcg gaagaagcgt cgtcgtag
1188

<210>    2
<211>    395
<212>    PRT
<213>    Arabidopsis thaliana

<400>    2
Met Val Gly Ser Arg Lys Arg Ser Ser Ala Lys Asn Arg Leu Val Ser
1               5                   10                  15

Ser Ser Thr Arg Ser Ser Gly Lys Asp Lys Val Ser Asp Ala Arg Lys
            20                  25                  30

Thr Gly Glu Ala Leu Val Gly Ser Arg Ile Arg Val Trp Trp Pro Met
        35                  40                  45

Asp Ser Lys Phe Thr Lys Gly Val Val Asp Ser Tyr Val Ser Ser Lys
    50                  55                  60

Lys Lys His Arg Val Phe Tyr Glu Asp Gly Asp Lys Glu Thr Leu Asp
65                  70                  75                  80

Leu Lys Lys Glu Arg Trp Glu Leu Ile Glu Glu Asp Asp Ala Glu Ser
                85                  90                  95

Glu Ser Asp Glu Ile Ser Leu Gln Glu Glu Ser Ala Gly Glu Ser Ser
            100                 105                 110

Glu Gly Thr Pro Glu Pro Pro Lys Gly Lys Gly Lys Ala Ser Leu Lys
        115                 120                 125

Gly Arg Thr Asp Glu Ala Met Pro Lys Lys Lys Gln Lys Ile Asp Ser
    130                 135                 140

Ser Ser Lys Ser Lys Ala Lys Glu Val Glu Lys Lys Ala Ser Lys Pro
145                 150                 155                 160

Glu Thr Glu Lys Asn Gly Lys Met Gly Asp Ile Gly Gly Lys Ile Val
                165                 170                 175

Ala Ala Ala Ser Arg Met Ser Glu Arg Phe Arg Ser Lys Gly Asn Val
            180                 185                 190

Asp Gln Lys Glu Thr Ser Lys Ala Ser Lys Lys Pro Lys Met Ser Ser

195                 200                 205

Lys Leu Thr Lys Arg Lys His Thr Asp Asp Gln Asp Glu Asp Glu Glu
    210                 215                 220

Ala Gly Asp Asp Ile Asp Thr Ser Ser Glu Glu Ala Lys Pro Lys Val
225                 230                 235                 240

Leu Lys Ser Cys Asn Ser Asn Ala Asp Glu Val Ala Glu Asn Ser Ser
                245                 250                 255

Asp Glu Asp Glu Pro Lys Val Leu Lys Thr Asn Asn Ser Lys Ala Asp
            260                 265                 270

Lys Asp Glu Asp Glu Glu Glu Asn Glu Thr Ser Asp Asp Glu Ala Glu
        275                 280                 285

Pro Lys Ala Leu Lys Leu Ser Asn Ser Asn Ser Asp Asn Gly Glu Asn
    290                 295                 300

Asn Ser Ser Asp Asp Glu Lys Glu Ile Thr Ile Ser Lys Ile Thr Ser
305                 310                 315                 320

Lys Lys Ile Lys Ser Asn Thr Ala Asp Glu Glu Asn Gly Asp Asn Glu
                325                 330                 335

Asp Gly Glu Lys Ala Val Asp Glu Met Ser Asp Gly Glu Pro Leu Val
            340                 345                 350

Ser Phe Leu Lys Lys Ser Pro Glu Gly Ile Asp Ala Lys Arg Lys Lys
        355                 360                 365

Met Lys Gly Lys Lys Glu Glu Glu Glu Glu Gly Glu Glu Asn Ala
    370                 375                 380

Gly Lys Asp Thr Lys Ala Glu Glu Ala Ser Ser
385                 390                 395


<210>   3
<211>   15
<212>   DNA
<213>   Artificial Sequence

<220>
<223>   primer


<400>   3
ntcgastwts gwgtt
15


<210>   4
<211>   16
<212>   DNA
<213>   Artificial Sequence

<220>
<223>   primer

<400>    4
ngtccaswga nawgaa
16


<210>    5
<211>    16
<212>    DNA
<213>    Artificial Sequence

<220>
<223>    primer


<400>    5
wgtgnagwan canaga
16


<210>    6
<211>    22
<212>    DNA
<213>    Artificial Sequence

<220>
<223>    primer


<400>    6
ccgagcggcg aactaataac gt
22


<210>    7
<211>    22
<212>    DNA
<213>    Artificial Sequence

<220>
<223>    primer


<400>    7
gaaagttacg ggcaccattc aa
22


<210>    8
<211>    22
<212>    DNA
<213>    Artificial Sequence

<220>
<223>    primer


<400>    8
gaagtgcagg tcaaaccttg ac

22

<210> 9
<211> 26
<212> DNA
<213> Artificial Sequence

<220>
<223> primer

<400> 9
gctagcatgg ttggttctag gaagcg
26

<210> 10
<211> 26
<212> DNA
<213> Artificial Sequence

<220>
<223> primer

<400> 10
actagtgtac gacgacgctt cttccg
26

## Claims

1. A polypeptide having a function for decaying flowering and/or suppressing growth in plants, selected from the group consisting of:
(a) a polypeptide having an entire amino acid sequence of SEQ. ID. NO: 2;
(b) a polypeptide containing a substantial part of the amino acid sequence of SEQ. ID. NO: 2; and
(c) a polypeptide substantially similar to that of (a) or (b).

2. The polypeptide of claim 1, wherein the polypeptide substantially similar to that of (a) or (b) shares a sequence homology of 80% or higher with the amino acid sequence of SEQ ID NO: 2.

3. The polypeptide of claim 1, wherein the polypeptide substantially similar to that of (a) or (b) shares a sequence horology of 90% or higher with the amino acid sequence of SEQ ID NO: 2.

4. The polypeptide of claim 1, wherein the polypeptide substantially similar to that of (a) or (b) shares a sequence homology of 95% or higher with the amino acid sequence of SEQ ID NO: 2.

5. A polynucleotide, encoding the polypeptide of any one of claims 1 to 4.

6. A polynucleotide, selected from the group consisting of:
(a) a polynucleotide containing a part of the nucleotide sequence of SEQ ID NO: 1; and
(b) a polynucleotide substantially similar to a part of the nucleotide sequence of SEQ ID NO: 1.

7. The polynucleotide of claim 6 for use in identifying or isolating a gene functioning to induce a delay in flowering and/or growth suppression in a plant.

8. A method for producing a plant having a delayed flowering and/or suppressed growth phenotype, comprising:
(I) over-expressing a gene having the nucleotide sequence of SEQ ID NO: 1 or a nucleotide sequence similar to that of SEQ ID NO: 1; and
(II) selecting the plant in which delayed flowering and/or suppressed growth phenotype is induced.

9. The method of claim 8, wherein the nucleotide sequence similar to that of SEQ ID NO: 1 shares a sequence horology of 30% or higher with the nucleotide sequence of SEQ ID NO: 1.

10. The method of claim 8, wherein the overexpression step (I) comprises transforming the polynucleotide of claim 5 into a plant.

11. The method of claim 10, wherein the transformation step comprises (a) inserting a polynucleotide encoding the polypeptide of claim 5 into an expression vector containing a regulatory nucleotide sequence to construct a recombinant expression vector and (b) introducing the recombinant vector into a host plant.

12. The method of claim 10, wherein the transformation step comprises inserting a polypeptide encoding the polypeptide of claim 5 into an expression vector to construct a recombinant expression vector, transforming an Agrobacterium sp. with the recombinant expression vector, and transfecting the transformed Agrobacterium sp. into a plant.

13. The method of claim 10, wherein the transformation step comprises transforming an Agrobacterium sp with a recombinant vector tarrying a gene comprised of the nucleotide sequence of SEQ ID NO: 1, and transecting the transformed Agrobacterium sp. into a plant.

14. A method for producing a plant having a suppressed growth phenotype, comprising:
(I) overexpressing a gene having the nucleotide sequence of SEQ ID NO: 1 or a nucleotide sequence similar to that of SEQ ID NO: 1; and
(II) selecting a plans in which a delayed flowering and/or suppressed growth phenotype is induced.

15. The method of claim 14, wherein the nucleotide sequence similar to that of SEQ ID NO: 1 shares a sequence homology of 90% or higher with the nucleotide sequence of SEQ ID NO: 1.

16. The method of claim 14, wherein the overexpression step (I) comprised transforming the polynucleotide of claim 5 into a plant.

17. The method of clam 14, wherein the transformation step comprises (a) inserting a polynucleotide encoding the polypeptide of claim 5 into an expression vector containing a regulatory nucleotide sequence to construct a recombinant expression vector and (b) introducing the recombinant vector into a host plant.

18. The method of claim 14, wherein the transformation step comprises inserting a polypeptide encoding the polypeptide of claim 5 into an expression vector to construct a recombinant expression vector, transforming an Agrobacterium sp. with the recombinant expression vector, and transfecting the transformed Agrobacterium sp. into a plant.

19. The method of claim 14, wherein the transformation step comprises transforming an Agrobacterium sp with a recombinant vector carrying a gene comprised of the nucleotide sequence of SEQ ID NO: 1, and transfecting the transformed Agrobacterium sp. into a plant.

20. A method for selecting a transgenic plant, comprising:
(I) transforming a plant with an expression vector carrying a target gene, the polynucleotide of claim 5, and a regulator nucleotide, and
(II) discriminating a flowering delay and/or growth suppression-induced plant variant.

21. The method of claim 20, wherein the transformation step (I) comprises transforming the expression vector into Agrobacterium sp. and transecting the transformed Agrobacterium sp. into the plant.

22. The method of claim 20, wherein the transformation step (I) comprises transforming an Agrobacterium sp with a recombinant vector carrying a gene comprised of the nucleotide sequence of SEQ ID NO: 1, and transfecting the transformed Agrobacterium sp. into a plant.

23. A plant showing a delayed flowering phenotype, produced by the method of any one of claims 8 to 13.

24. A plant showing a suppressed growth phenotype, produced by the method of any one of claims 14 to 19.
